# EUROPEAN PATENT SPECIFICATION

(11) **EP 1 795 197 B1**
(45) Date of publication and mention of the grant of the patent: **23.01.2013**
(21) Application number: 06125456.1
(22) Date of filing: 05.12.2006
(51) Int. Cl.: A61K 31/546, A61P 31/04

(54) **Pharmaceutical compositions comprising an antibiotic**
Pharmazeutische Zusammensetzung mit einem Antibiotikum
Compositions pharmaceutiques comprenant un antibiotique

(30) Priority: 07.12.2005 US 295929
(43) Date of publication of application: 13.06.2007
(73) Proprietor: SANDOZ AG, 4056 Basel (CH)
(72) Inventor: Jennewein, Herwig, 6067 Absam (AT)
(74) Representative: Wichmann, Hendrik

(56) References cited:
- EP-A- 1 609 793
- WO-A-2004/104010
- US-A1- 2003 204 082
- US-A1- 2004 242 556
- US-A1- 2005 137 182
- US-A1- 2005 245 738

## Description

### Field of the Invention

The present invention relates to cefdinir. More particularly to pharmaceutical formulations comprising cefdinir in a defined polymorphic form and processes for the preparation thereof. Furthermore, the present invention relates to processes to keep cefdinir in a defined polymorphic form.

### Background of the Invention

Cefdinir, 7-[2-(2-aminothiazol-4-yl)-2-hydroxyiminoacetamido]-3-vinyl-3-cephem-4-carboxylic acid, is a cephalosporin known to have high antibiotic activity and is for example described in US 4,559,334.
A crystalline form of cefdinir, identified as form A, is claimed in US 4,935,507. Briefly, crystalline cefdinir is described to be obtainable by crystallization from acidified solutions at ambient temperatures (see e.g. Example 2 in column 12). Form A is comprised in the commercially available form of cefdinir, e.g. as sold under the trademark Omnicef^{®}. Crystalline form B of cefdinir has been described, for example, in US 2003/0204082 in example 1 on page 1.

Different crystal forms of one compound may interconvert, that is under certain conditions one crystal form with favorable characteristics may convert to another crystal form with possibly less favorable characteristics. There is thus a need for pharmaceutical compositions comprising cefdinir in that defined polymorphic state which shows favorable characteristics.

### Summary of the Invention

The present invention provides pharmaceutical compositions comprising cefdinir, wherein more than 95% of the cefdinir is stably present as crystalline form B of cefdinir and wherein the equilibrium relative humidity of the composition is below 65% when measured at 25°C +1-1°C.

Additionally, the present invention provides a container comprising a pharmaceutical composition of the invention and a means to keep the equilibrium relative humidity of the composition at below 65%.

The present invention also provides processes for preparing a pharmaceutical composition of the invention.

### Brief Description of the Drawing

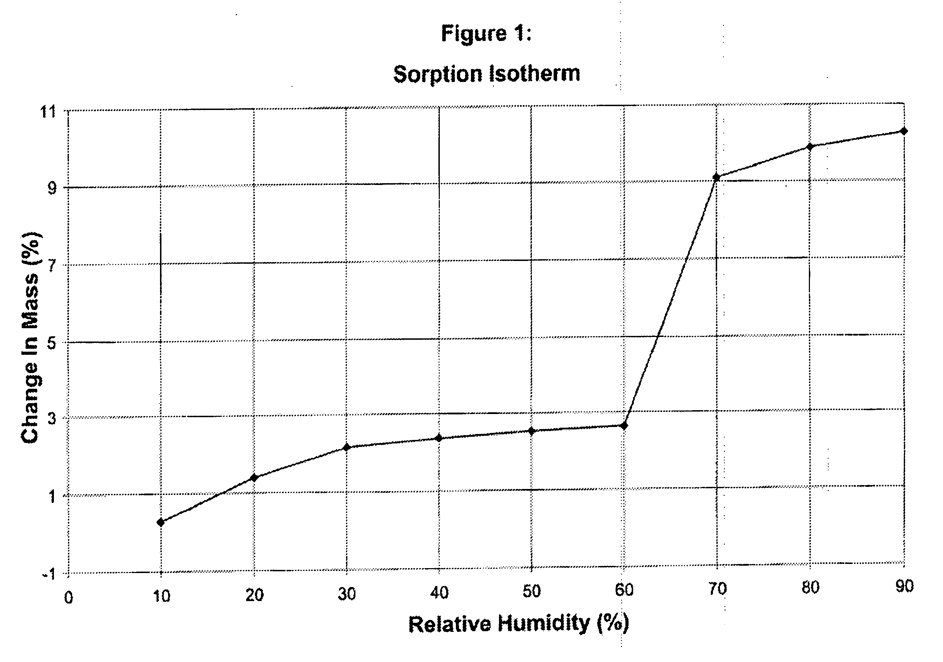

Figure 1 shows the sorption isotherm of a test sample of crystalline form B of cefdinir which is measured by using a Dynamic Vapour Sorption apparatus such as commercially available as type DVS 1 from Surface Measurement Systems Ltd., UK. Starting from a relative humidity of 10%, the change in mass of the test sample is measured by stepwise adjusting the ambient relative humidity within the measuring apparatus to target values of 10%, 20%, 30%, 40%, 50%, 60%, 70%, 80% and 90%, and by weighing at each step the mass of the test sample when said weight has reached a stable value at a given relative humidity. These values are expressed as Change in Mass in % versus the dry weight of the test sample (ordinate) and are plotted against the relative humidity in % (abscissa). The dry weight of the test sample is determined at a relative humidity of 0%.

### Detailed Description of the Invention

Crystalline form B of cefdinir as defined for the purpose of the present invention is a crystalline form of cefdinir wherein the X-ray powder diffraction (XRPD) pattern as obtained with Cu Kd radiation comprises characteristic peaks at the 2 θ diffraction angles of 11.7 ± 0.2, 16.2 ± 0.3, 21.2 ± 0.3, 22.3 ± 0.1 and 24.4 ± 0.1, preferably also 26.4 ± 0.2. Crystalline form B can have a water content of below 8%. Crystalline form B with a water content of from 6% to 7% can be obtained according to the examples 2, 3 and 4 of US 2005/0245738. Crystalline form B with a water content of from 5.5% to 7% can be obtained according to the examples 2 and 3 of US 2005/0137182. Preferably, cefdinir is obtained by following example 1 from US 2003/0204082. Apparently a form which is also a preferred cefdinir form B can also be obtained by following example 1 of WO 2004/104010.

It has now been found that crystalline form B of cefdinir transforms into a different polymorphic form wherein cefdinir exhibits reduced stability when form B crystals of cefdinir are exposed to conditions with higher than 65% relative humidity. Usually minutes of exposure to high humidity and seconds of exposure to water, such as within an suspension, are sufficient for this transformation. Such conditions are for example found during pharmaceutical production in countries with a humid climate.

The above described transformation of crystalline form B of cefdinir into a different polymorphic form can be concluded from Figure 1 which shows that the mass of a test sample of crystalline form B of cefdinir changes when exposed to increasing values of relative humidity; in particular, a drastic change in water content observed at a relative humidity of about 60% to 70% seems to reflect said transformation of crystalline from B of cefdinir to a different polymorphic form. The X-ray powder diffraction pattern also changed at high relative humidity.

The present invention therefore relates to a pharmaceutical composition comprising cefdinir wherein more than 95% of the cefdinir present in said composition is stably present as crystalline form B of cefdinir, more preferably to pharmaceutical compositions comprising cefdinir wherein the cefdinir present in said composition does not show XRPD-peaks at the diffraction angles [2 θ (°)] of about 14.7, 17.8 or 10.6. In particular, the present invention relates to such pharmaceutical compositions wherein crystalline form B of cefdinir is the only detectable crystalline from of cefdinir.

"Stably present" as defined herein means that even after storage of the pharmaceutical composition for 180 days, and preferably even after storage for two years, the crystalline from B of cefdinir initially comprised in the pharmaceutical composition is still present as crystalline form B of cefdinir after storage for the indicated period. Such compositions can be produced by avoiding humid conditions, such as high relative humidity of the air, during the formulation steps. Furthermore, the above-identified humid conditions are to be avoided during storage in order to preserve the pharmaceutical composition of the invention.

In a preferred embodiment the pharmaceutical composition of the invention comprises the crystalline form B of cefdinir as the only detectable form of cefdinir. Analysis of the polymorphic state of cefdinir in a pharmaceutical composition can be performed by any suitable method known in the art, for example by XRPD.

The pharmaceutical composition of the invention exhibits an equilibrium relative humidity of below 65% when measured at 25°C +/- 1°C, preferably of from 3% to 60%, more preferably of from 10% to 60%, preferably from 15% to 60%, in particular more preferably of from 15% to 30% or from 30% to 60%, for at least 180 days, preferably for at least two years.

The equilibrium relative humidity of the pharmaceutical compositions or of crystalline form B of cefdinir as herein described is measured by determining the relative humidity in % in the air above a test sample, e.g. a pharmaceutical composition of the invention, after establishment of a humidity equilibrium in a closed system at a constant temperature according to the following method: the equipment used is the commercially available measuring chamber Rotronic AW-VC comprising a hygrometer of the type BT-RS1. The test sample, e.g. a pharmaceutical composition of the invention, is filled into a sampling dish which is placed into the measuring chamber which has been thermostated to a temperature of 25 +/- 1°C, said chamber is subsequently closed and sealed. After establishment of an equilibrium of the relative humidity which state is typically shown by the disappearance of a trend indication, the value of the relative humidity in % is read from the hygrometer. Relative humidity is defined as the equilibrium relative humidity of the pharmaceutical compositions as measured as herein described. Filling of the chamber is to be performed in such a way as to provide complete filling of said chamber according to the instructions of the manufacturers. In case the test sample is a powder or granules for oral suspension, or a liquid suspension, said sample is directly placed into the above mentioned sampling dish. In case the test sample is a capsule, the appropriate number of capsules are opened and their contents is filled into the sampling dish. In case the test sample is a tablet, the appropriate number of tablets is crushed by using a mortar, and filled into the sampling dish. In cases where the equilibrium humidity is expected to be below 20%, the above described preparation of the test samples before measurement and the measurement itself as herein described is to be performed in a glove box being equipped with a hygrometer wherein a relative humidity of about 5% is to be established by e.g. flushing with dried air or nitrogen. The above described method for measurement of the equilibrium relative humidity of the pharmaceutical compositions of the invention is herein also called ERH method.

The pharmaceutical composition of the present invention is preferably stored in a relatively dry environment, and preferably it is to be assured that the storage environment remains relatively dry during the lifetime of the pharmaceutical composition.

The invention therefore also relates to a container comprising a pharmaceutical composition of the invention, which container is capable to keep the equilibrium relative humidity of the composition at below 65%, preferably at from 10% to 60%, more preferably at from 15% to 60%, for at least 180 days, more preferably for at least two years. This can be achieved, for example, by use of a tightly sealed container, or by equipping the container with a means to keep the composition relatively dry.

Such a drying means may be, for example, desiccant bags, e.g. as commercially available under the trade name MINIPAX and containing 2 g of molecular sieve 4 Angstrom; or desiccant canisters, e.g. as available under the trade name SORBIT and containing 1 g Silicagel; desiccant capsules, e.g. as available under the trade name DRICAP, and containing 0.9 g Silicagel, or desiccant stoppers containing 2 g Silicagel.

The products or intermediate products obtained in the various steps of herein described processes are preferably stored at an environmental relative humidity of below 65%. Said products may thus be stored in aluminium barrels or drums, in so-called Nirosta® drums, such as commercially available as Müller® drums. Said drums may be made gas-tight, e.g. air-tight by applying a sealing means, such as sealing rings to the lid thereof. Said products may also be stored in containers made of aluminium or Nirosta®-material as mentioned above whereof the closures or lids are provided with a sealing means, such as a sealing ring.

The pharmaceutical compositions of the invention are preferably packaged or filled into containers as herein described at an environmental relative humidity of below 65%, preferably at from 10% to 60%. Subsequently, said containers are tightly closed as herein described. Preferably, said containers are used for stable storage of the pharmaceutical compositions of the invention, for example at room temperature, such as at a temperature of about 20°C to 30°C, e.g. at about 25°C, for a prolonged period, e.g. for at least 6 months, preferably at least about 24 months, e.g. for up to at least 24 months, e.g. for up to at least about 30 months, such as for up to about 60 months.

A preferred container is a bottle, e.g. a glass or plastic bottle, e.g. a polyethylene bottles, such as known as securitainer, having e.g. a screw closure, or is a blister, e.g. an aluminium blister or strip, e.g. a blister consisting of 2 aluminium foils or strips, or a blister comprising an Aclar® foil and an aluminium cover foil, or may be any other suitable container. More preferably said container is a gas-tight container, such as an air-tight container.

Preferred containers are glass or plastic bottles sealed with an aluminium membrane, alu-alu-blisters or strips, or blisters comprising an Aclar® foil and an aluminium cover foil.
The container according to the invention is obtained by filling the pharmaceutical compositions of the invention into said container under the conditions as herein described.

Therefore, the present invention also relates to a container as described above, wherein the container in combination with the drying means is capable of maintaining the equilibrium relative humidity of the pharmaceutical composition of the invention therein comprised at below 65%, preferably at from 10% to 60%, for at least 6 months, preferably for at least two years. In a preferred embodiment the container further encloses a gaseous atmosphere with a relative humidity of below 65%, preferably of from 10% to 60%. Equipping the container with a dry gaseous atmosphere, for example dry air or dry nitrogen gas, can be performed as known in the art.

Preferred combinations of container and drying means are aluminium-foil-sealed polyethylene-bottles (PE-bottles) containing desiccant capsules and/or canisters or glass bottles with desiccant stoppers.

As mentioned above, special care as to the relative environmental humidity and as to the equilibrium relative humidity of the composition has to be taken during the production of pharmaceutical compositions of the invention. Therefore, the present invention also relates to a process for preparing a pharmaceutical composition of the invention comprising the steps of
a) mixing the crystalline form B of cefdinir with one or more pharmaceutically acceptable excipients at a relative humidity of below 65%, preferably at from 10% to 60%;
b) optionally granulating the mixture obtained in step a) at a relative humidity of below 65%, preferably at from 10% to 60%; and
c) further processing the mixture obtained in step a) or the granulate obtained in step b) at a relative humidity of below 65%, preferably at from 10% to 60%, to obtain a pharmaceutical composition of the invention. It is preferred that the obtained pharmaceutical composition of the invention exhibits an equilibrium relative humidity of below 65%, preferably of from 10% to 60%, more preferably of from 15% to 30% or of from 30% to 60%.

The mixture obtained from step a) or the granulate obtained from step b) as described above is preferably processed into an oral dosage form, like a capsule or a tablet, or granules for oral suspension, or a powder for oral suspension.

In a preferred embodiment, the obtained pharmaceutical composition having an equilibrium relative humidity of below about 65%, preferably of from 10% to 60%, is filled into a container capable of maintaining the equilibrium relative humidity of the pharmaceutical composition at below 65%, preferably at from 10% to 60%, for at least 6 months, for examples the containers mentioned above, which may optionally further comprise a drying means sufficient to maintain the equilibrium relative humidity of the pharmaceutical composition at below 65%, preferably at from 10% to 60%.

As explained above, proper storage conditions for the pharmaceutical compositions of the invention are important for maintaining the compositions in the desired form. Thus, the present invention further relates to the use of a container capable of maintaining a gaseous atmosphere at a relative humidity of below 65%, preferably at from 10% to 60%, for at least 6 months for storage of a pharmaceutical composition of the invention. Further, the present invention discloses the use of a gaseous atmosphere having a relative humidity of below 65%, preferably at from 10% to 60%, to stabilize the crystalline form B of cefdinir.

The pharmaceutical compositions of the invention may further comprise one or more pharmaceutically acceptable excipients which are preferably selected from the group consisting of fillers, sweeteners, buffering agents, glidants, flowing agents, flavouring agents, lubricants, preservatives, surfactants, wetting agents, binders, disintegrants and thickeners. Other excipients known in the field of pharmaceutical compositions may also be used. Furthermore, the pharmaceutical compositions may comprise a combination of 2 or more excipients also within one of the members of the above mentioned group. Preferably, the fillers are also sweeteners.

Preferred fillers are sucrose, mannitol and cellulose. Preferred sweeteners are sucrose and Aspartame. Preferred buffering agents are citric acid and sodium citrate. A preferred glidant or flowing agent is colloidal silica, e.g. Aerosil^{®}. Preferred flavours are strawberry and raspberry. A preferred lubricant and/or wetting agent is magnesium stearate. A preferred preservative is sodium benzoate. A preferred surfactant is sodium lauryl sulfate. Preferred disintegrants are cross-linked polyvinylpyrrolidones, e.g. crospovidones, cross-linked sodium carboxymethylcellulose, e.g.,croscarmellose sodium and calcium-carmellose. Preferred thickeners are xanthan gum and guar gum. Examples of suitable binders include starches and modified starches, e.g., pregelatinized starch, celluloses, e.g. hydroxypropykmethylcellulose, hydroxypropyl cellulose, hydroxyethyl cellulose and polyvinyl pyrrolidone, e.g., Povidone.

Two particularly preferred pharmaceutical compositions of the invention are described in the exemplifying section and were prepared and stored at a relative humidity of below 65%, preferably of from 10% to 60%.

Optionally, the pharmaceutical compositions of the invention may comprise a coating which comprises as film-forming agent such as e.g. cellulose and derivatives thereof, hydroxypropylmethylcellulose, polyvinylpyrrolidone, or enteric film-forming agents such as cellulose phthalates, poly(meth)acrylates and polyvinyl acetate-phthalate.

The pharmaceutical compositions of the invention are preferably forms for oral administration, and may be in the form of a tablet, a capsule, a caplet, granules for oral suspension, a powder for oral suspension, a dispersible tablet, or in the form of a liquid suspension.

After the pharmaceutical compositions of the invention have been filled into the herein mentioned containers, said containers are preferably tightly closed, e.g. tightly or hermetically sealed, e.g. in a way to prevent any gaseous atmosphere from diffusing through the walls and/or closure of said containers. Methods of tightly sealing and/or closing said containers are known, such as sealing of glass or plastic bottles by applying an aluminium membrane to the bottle opening of said bottle by induction sealing and by applying a closure, e.g. a screw closure, or such as sealing of alu-alu blisters or strips, of blisters comprising an Aclar® foil and an aluminium cover foil by heat sealing according, e.g. analogously to known methods.

Within said preferably tightly sealed container comprising a gaseous atmosphere, preferably air, having a relative humidity of below 65%, preferably of from 10% to 60%, is maintained stable for at least 6 months, preferably at least 24 months. Thereby, the crystalline form B of cefdinir comprised in the pharmaceutical compositions of the invention is stabilized in its form as herein defined over a period of at least 6 months, preferably for at least 24 months, such as for about 36 months.

Thus, the present invention also provides the use of a container capable of maintaining a gaseous atmosphere at a relative humidity of below 65%, preferably of from 10% to 60%, for at least 6 months for storage of a pharmaceutical composition of the invention.

In a further aspect, the invention provides a pharmaceutical composition comprising crystalline form B of cefdinir being obtainable by the processes as herein described preferably under the conditions as herein described with regard to starting material, relative humidity and further processing. Furthermore, said pharmaceutical compositions may be packaged and/or stored under the conditions and for the time period as herein described.

The temperature applied during the herein described processes is preferably room temperature, e.g. is a temperature of about 20°C to about 30°C, such as about 25°C.

The pharmaceutical compositions of the invention typically comprise a therapeutically effective amount of crystalline form B of cefdinir. The pharmaceutical compositions of the invention may comprise for example units doses of about 1 mg to about 2000 mg of cefdinir, preferably of from 100 mg to 1000 mg of cefdinir.

The pharmaceutical compositions of the invention may be used as a medicament, e.g. for the treatment of infectious diseases caused by germs which are sensitive to cefdinir. Said diseases are for example upper and lower respiratory tract infections and infections of skin and soft tissues.

In another aspect of the invention, pharmaceutical compositions of the invention may be used for the preparation of a medicament for the treatment of the herein mentioned infectious diseases.

There is also provided a method of treating infectious diseases caused by germs sensitive to cefdinir by administering the pharmaceutical compositions of the invention to a mammal, e.g. a human patient, in need thereof.

The present invention is further illustrated by the following examples, which are not to be construed to be in any way limiting to the present invention.

### Example 1

### Preparation of a dry suspension comprising crystalline form B of cefdinir

| Ingredient | Amount per unit dose* | |
|---|---|---|
| Cefdinir Monohydrate | 261.5 mg | (corresponding to 250 mg cefdinir) |
| Sucrose | 2671 mg | |
| Xanthan gum | 4 mg | |
| Guar gum | 8 mg | |
| Citric acid | 3 mg | |
| Sodium citrate | 3 mg | |
| Aerosil® | 10 mg | |
| Magnesium stearate | 15 mg | |
| Sodium benzoate | 5 mg | |
| Strawberry flavour | 20 mg | |

| | | |
|---|---|---|
| * unit dose per 5 ml of ready made suspension, i.e. dry suspension plus water added in an amount as to obtain 5 ml of suspension | | |

Cefdinir B is mixed with half of the indicated amount of the sucrose and of the magnesium stearate, and with the complete amount of the xanthan gum, guar gum and sodium benzoate. The obtained mixture is subsequently processed by dry granulation using a compactor. The resulting compacted scabs are broken and milled. The indicated amounts of citric acid, trisodium citate, Aerosil® and strawberry flavour and the remaining part of sucrose and magnesium stearate are added to the resulting granulate and thoroughly mixed. The resulting mixture is then filled into glass bottles in an amount corresponding to the desired number of unit doses. The filled bottles are subsequently closed tightly by applying an aluminium membrane onto the bottle opening by induction sealing and by applying a screw closure. The overall process takes place at about 45% relative humidity of the air.

### Example 2

### Preparation of capsules comprising crystalline form B of cefdinir

| Ingredient | Amount per capsule | |
|---|---|---|
| Cefdinir Monohydrate | 313.8 mg | (corresponding to 300 mg cefdinir) |
| Magnesium stearate | 5 mg | |
| Aerosil® | 1 mg | |
| Myrj® 52 | 2 mg | |
| Calcium-Carmellose | 58 mg | |

Cefdinir Form B is mixed with the complete amount of Aerosil®, calcium-carmellose and Myrj® 52 (polyoxyethylene-40-stearate) and with a first portion of magnesium stearate. The resulting mixture is subsequently compacted in a compactor, the obtained compacted scabs are broken and millled. The resulting granulate is subsequently mixed with the remaining portion of the magnesium stearate, and the obtained mixture is filled into hard gelatine capsules having the appropriate size. The capsules are subsequently filled into polyethylene (PE) bottles, and SORBIT desiccant canisters are added. Said PE bottles are then closed tightly by applying an aluminium membrane onto the bottle opening by induction sealing and by applying the screw closure. The overall process takes place at about 45% relative humidity of the air.

## Claims

1. A pharmaceutical composition comprising cefdinir, wherein more than 95% of the cefdinir is stably present as crystalline form B of cefdinir, wherein the X-ray powder diffraction pattern of said crystalline form B of cefdinir as obtained with Cu Ka radiation comprises characteristic peaks at the 2 θ diffraction angles of 11.7 ± 0.2, 16.2 ± 0.3, 21.2 ± 0.3, 22.3 ± 0.1 and 24.4 ± 0.1, and wherein the equilibrium relative humidity of the composition is below 65% when measured at 25°C +/- 1°C.

2. The pharmaceutical composition of claim 1, wherein the equilibrium relative humidity of the composition is from 10% to 60%.

3. A container comprising a pharmaceutical composition according to claim 1 and a means to keep the equilibrium relative humidity of the composition at below 65%.

4. The container of claim 3, wherein the container in combination with the means to keep the equilibrium relative humidity of the composition at below 65% is capable of maintaining the equilibrium relative humidity of the composition at below 65% for at least 6 months.

5. The container of claim 3, wherein the container further encloses a gaseous atmosphere with a relative humidity of below 65%.

6. A process for preparing a pharmaceutical composition according to claim 1 comprising the steps of
a) mixing the crystalline form B of cefdinir, wherein the X-ray powder diffraction pattern of said crystalline form B of cefdinir as obtained with Cu Kα radiation comprises characteristic peaks at the 2 θ diffraction angles of 11.7 ± 0.2, 16.2 ± 0.3, 21.2 ± 0.3, 22.3 ± 0.1 and 24.4 ± 0.1, with one or more pharmaceutically acceptable excipients at a relative humidity of below 65%;
b) optionally granulating the mixture obtained in step a) at a relative humidity of below 65%; and
c) further processing the mixture obtained in step a) or the granulate obtained in step b) at a relative humidity of below 65 % to obtain a pharmaceutical composition according to claim 1.

7. The process of claim 6 wherein the mixture or granulate is processed into an oral dosage form.

8. The process of claim 7 wherein the oral dosage form is a capsule or a tablet.

9. The process of any of claims 6 to 8 comprising the additional step of filling the obtained pharmaceutical composition having an equilibrium relative humidity of below 65% when measured at 25°C +/- 1°C into a container capable of maintaining the equilibrium relative humidity of the pharmaceutical composition at below 65% when measured at 25°C +/- 1°C for at least 6 months.

10. Use of a container capable of maintaining a gaseous atmosphere at a relative humidity of below 65% for at least 6 months for storage of a pharmaceutical composition according to claim 1.

## Patentansprüche

1. Pharmazeutische Zusammensetzung umfassend Cefdinir, wobei mehr als 95% des Cefdinirs als kristalline Form B von Cefdinir stabil vorliegt, wobei das Pulver-Röntgenbeugungsmuster der kristallinen Form B von Cefdinir wie mit Cu-Kα-Strahlung erhalten charakteristische Peaks bei den 2-θ-Beugungswinkeln von 11,7 ± 0,2, 16,2 ± 0,3, 21,2 ± 0,3, 22,3 ± 0,1 und 24,4 ± 0,1 umfasst, und wobei die relative Gleichgewichtsfeuchtigkeit der Zusammensetzung unterhalb 65% liegt, wenn bei 25 °C ± 1 °C gemessen.

2. Pharmazeutische Zusammensetzung nach Anspruch 1, wobei die relative Gleichgewichtsfeuchtigkeit der Zusammensetzung von 10% bis 60% liegt.

3. Behälter umfassend eine pharmazeutische Zusammensetzung gemäß Anspruch 1 und ein Mittel, um die relative Gleichgewichtsfeuchtigkeit der Zusammensetzung bei unterhalb 65% zu halten.

4. Behälter nach Anspruch 3, wobei der Behälter in Kombination mit den Mitteln, um die relative Gleichgewichtsfeuchtigkeit der Zusammensetzung bei unterhalb 65% zu halten, fähig ist, die relative Gleichgewichtsfeuchtigkeit der Zusammensetzung bei unterhalb 65% für mindestens 6 Monate zu halten.

5. Behälter nach Anspruch 3, wobei der Behälter des Weiteren eine gasförmige Atmosphäre mit einer relativen Feuchtigkeit von unterhalb 65% enthält.

6. Verfahren zur Herstellung einer pharmazeutischen Zusammensetzung gemäß Anspruch 1, umfassend die Schritte von
a) Mischen der kristallinen Form B von Cefdinir, wobei das Pulver-Röntgenbeugungsmuster der kristallinen Form B von Cefdinir wie erhalten mit Cu-Kα-Strahlung charakteristische Peaks an den 2-θ-Beugungswinkeln von 11,7 ± 0,2, 16,2 ± 0,3, 21,2 ± 0,3, 22,3 ± 0,1 und 24,4 ± 0,1 umfasst, mit einem oder mehreren pharmazeutisch verträglichen Hilfsstoffen bei einer relativen Feuchtigkeit von unterhalb 65%;
b) gegebenenfalls Granulieren der in Schritt a) erhaltenen Mischung, bei einer relativen Feuchtigkeit von unterhalb 65%; und
c) Weiterverarbeiten der in Schritt a) erhaltenen Mischung oder des in Schritt b) erhaltenen Granulats bei einer relativen Feuchtigkeit von unterhalb 65%, um eine pharmazeutische Zusammensetzung gemäß Anspruch 1 zu erhalten.

7. Verfahren nach Anspruch 6, wobei die Mischung oder das Granulat in eine orale Arzneiform verarbeitet wird.

8. Verfahren nach Anspruch 7, wobei die orale Arzneiform eine Kapsel oder eine Tablette ist.

9. Verfahren nach einem beliebigen der Ansprüche 6 bis 8 umfassend den zusätzlichen Schritt des Füllens der erhaltenen pharmazeutischen Zusammensetzung mit einer relativen Gleichgewichtsfeuchtigkeit von unterhalb 65%, wenn gemessen bei 25 °C ± 1 °C, in einen Behälter, der zum Beibehalten der relativen Gleichgewichtsfeuchtigkeit der pharmazeutischen Zusammensetzung bei unterhalb 65%, wenn bei 25 °C ± 1 °C gemessen, für mindestens 6 Monate fähig ist.

10. Verwendung eines Behälters, der fähig ist, für mindestens 6 Monate eine gasförmige Atmosphäre mit einer relativen Feuchtigkeit von unterhalb 65% zu halten, zur Lagerung einer pharmazeutischen Zusammensetzung gemäß Anspruch 1.

## Revendications

1. Composition pharmaceutique comprenant du céfdinir, dans laquelle plus de 95% du céfdinir est présent de façon stable sous la forme cristalline B du céfdinir, dans laquelle le schéma de diffraction des rayons X sur poudre de ladite forme cristalline B du céfdinir tel qu'obtenu avec un rayonnement Kα de Cu comprend les pics caractéristiques aux angles de diffraction 2 θ de 11,7 ± 0,2, 16,2 ± 0,3, 21,2 ± 0,3, 22,3 ± 0,1 et 24,4 ± 0,1, et dans laquelle l'humidité relative à l'équilibre de la composition est inférieure à 65% lorsqu'elle est mesurée à 25°C +/- 1°C.

2. Composition pharmaceutique selon la revendication 1, dans laquelle l'humidité relative à l'équilibre de la composition est de 10% à 60%.

3. Récipient comprenant une composition pharmaceutique selon la revendication 1 et un moyen pour maintenir l'humidité relative à l'équilibre de la composition au-dessous de 65%.

4. Récipient selon la revendication 3, dans lequel le récipient en combinaison avec le moyen pour maintenir l'humidité relative à l'équilibre de la composition au-dessous de 65% est capable de maintenir l'humidité relative à l'équilibre de la composition au-dessous de 65% pendant au moins 6 mois.

5. Récipient selon la revendication 3, dans lequel le récipient renferme en outre une atmosphère gazeuse avec une humidité relative inférieure à 65%.

6. Procédé de préparation d'une composition pharmaceutique selon la revendication 1, comprenant les étapes consistant à
a) mélanger la forme cristalline B du céfdinir, dans laquelle le schéma de diffraction des rayons X sur poudre de ladite forme cristalline B du céfdinir tel qu'obtenu avec un rayonnement Kα de Cu comprend les pics caractéristiques aux angles de diffraction 2 θ de 11,7 ± 0,2, 16,2 ± 0,3, 21,2 ± 0,3, 22,3 ± 0,1 et 24,4 ± 0,1, avec un ou plusieurs excipients pharmaceutiquement acceptables à une humidité relative inférieure à 65% ;
b) éventuellement la granulation du mélange obtenu à l'étape a) à une humidité relative inférieure à 65% ; et
c) en outre le traitement du mélange obtenu à l'étape a) ou du granulé obtenu à l'étape b) à une humidité relative inférieure à 65% pour obtenir une composition pharmaceutique selon la revendication 1.

7. Procédé selon la revendication 6, dans lequel le mélange ou le granulé est transformé en une forme posologique orale.

8. Procédé selon la revendication 7, dans lequel la forme posologique orale est une gélule ou un comprimé.

9. Procédé selon l'une quelconque des revendications 6 à 8, comprenant l'étape supplémentaire de remplissage de la composition pharmaceutique obtenue ayant une humidité relative à l'équilibre inférieure à 65% lorsqu'elle est mesurée à 25°C +/- 1°C dans un récipient capable de maintenir l'humidité relative à l'équilibre de la composition pharmaceutique au-dessous de 65% lorsqu'elle est mesurée à 25°C +/- 1°C pendant au moins 6 mois.

10. Utilisation d'un récipient capable de maintenir une atmosphère gazeuse à une humidité relative inférieure à 65% pendant au moins 6 mois pour le stockage d'une composition pharmaceutique selon la revendication 1.
